# EUROPÄISCHE PATENTSCHRIFT

(11) **EP 3 334 359 B1**
(45) Veröffentlichungstag und Bekanntmachung des Hinweises auf die Patenterteilung: **13.03.2019**
(21) Anmeldenummer: 17751330.6
(22) Anmeldetag: 31.07.2017
(51) Int. Cl.: A61B 18/12, A61B 18/14

(54) **VERFAHREN UND VORRICHTUNG ZUR STEUERUNG DER ENERGIEZUFUHR ZU EINEM MEDIZINISCHEN INSTRUMENT**
METHOD AND DEVICE FOR CONTROLLING THE ENERGY SUPPLY TO A MEDICAL INSTRUMENT
PROCÉDÉ ET DISPOSITIF POUR COMMANDER L'ALIMENTATION EN ÉNERGIE D'UN INSTRUMENT MÉDICAL

(30) Priorität: 05.08.2016 DE 102016114537
(43) Veröffentlichungstag der Anmeldung: 20.06.2018
(73) Patentinhaber: Aesculap AG, 78532 Tuttlingen (DE)
(72) Erfinder: WALBERG, Erik, 78532 Tuttlingen (DE); EICK, Stefan, 78532 Tuttlingen (DE); KELLER, Anton, 78589 Dürbheim (DE)
(74) Vertreter: Winter, Brandl, Fürniss, Hübner, Röss, Kaiser, Polte - Partnerschaft mbB
(86) Internationale Anmeldenummer: PCT/EP2017/069296
(87) Internationale Veröffentlichungsnummer: WO 2018/024665

(56) Entgegenhaltungen:
- EP-A1- 2 792 326
- US-A1- 2003 176 778
- US-A1- 2009 062 786
- US-A1- 2016 174 819

## Beschreibung

Die Erfindung betrifft ein Verfahren und eine Vorrichtung zur Steuerung einer Energiezufuhr zu einem medizinischen Instrument, und bezieht sich insbesondere auf ein Verfahren und eine Einrichtung zur Auswahl einer von Betriebsarten einer Energieversorgungsvorrichtung eines medizinischen Instruments bei einem medizinischen Gerät oder System zur bipolaren Gefäßversiegelung.

In etwa der offenen und laparoskopischen Chirurgie sind beispielsweise in den chirurgischen Disziplinen der Allgemeinchirurgie, der Gynäkologie, der Urologie und der Thoraxchirurgie Geräte oder Systeme zur bipolaren Gefäßversiegelung bekannt.

Derartige bekannte Geräte und Systeme können bipolare Gefäßversiegelungsinstrumente, welche an einem distalen Ende über einen Schneide- und Versiegelungsabschnitt verfügen, mittels dem ein Chirurg Schnitte in Gewebeteilen und an Gefäßen ausführen und sodann Schnittbereiche und/oder Gefäße verschließen oder versiegeln kann, und einen Hochfrequenz (HF)-Generator zur Versorgung der Instrumente mit Energie beinhalten.

Bislang kann dabei eine Grundeinstellung, beispielsweise eine Betriebsartenwahl, unabhängig von einem angeschlossenen Instrument an dem Hochfrequenzgenerator vorgenommen und eine Energiezufuhr zu dem Instrument sodann über an dem Instrument angeordnete Bedienelemente, wie beispielsweise Schalteinrichtungen oder Taster, begonnen und beendet werden. In der Praxis wählt beispielsweise vor Beginn eines Eingriffs ein Chirurg an dem Hochfrequenz-Generator eine von möglichen Betriebsarten aus, und liefert sodann der Hochfrequenz-Generator eine der gewählten Betriebsart entsprechende Energie an den Versiegelungsabschnitt des mit dem Hochfrequenz-Generator verbundenen Instruments, nachdem und solange der Chirurg zugeordnete Bedienelemente an dem Instrument betätigt.

In einer bekannten Ausführungsform kann beispielsweise ein bekannter Hochfrequenz-Generator zwei auswählbare Versiegelungsprozess-Betriebsarten haben, spezieller eine erste, normale oder Standard-Betriebsart, welche eine kürzer versiegelnde Versiegelungsbetriebsart sein kann, und eine als Plus-Betriebsart bezeichenbare zweite Betriebsart, welche eine länger versiegelnde Versiegelungsbetriebsart sein kann. Ferner kann ein Handstück ein Bedienelement, beispielsweise einen Taster, aufweisen, welcher den Beginn und das Ende der Energiezufuhr steuert.
EP2792326 offenbart eine Energieversorgungseinrichtung und ein Instrument mit mehreren Schaltern.

Nachteilig hierbei ist bislang, dass das Bedienelement nur die an dem Generator ausgewählte Energiezufuhrbetriebsart, d.h. die erste oder die zweite Betriebsart, triggert bzw. auslöst und/oder anhält.

Daher erfordern es sämtliche bislang bekannten Instrumente, einen Energiezufuhrschalter für die Dauer des Versiegelungsprozesses gedrückt zu halten. Wird der Energiezufuhrschalter während des Versiegelungsprozesses losgelassen, wird der Versiegelungsprozess unterbrochen oder angehalten. Außerdem ist bei sämtlichen bislang bekannten Instrumenten für ein Bedienelement an dem Instrument oder Handstück nur eine Energiezufuhrbetriebsart verfügbar, da die Grundeinstellung oder Voreinstellung an dem leitungsverbundenen Hochfrequenz-Generator vorgenommen werden muss und/oder nur an diesem geändert werden kann.

Zur Umgehung dieser Nachteile kann im Stand der Technik zwar entweder ein zusätzliches dediziertes Bedienelement (Schalter) an dem Instrument vorgesehen sein, mittels dem die Betriebsart des Hochfrequenz-Generators geändert werden kann. Erkauft wird diese Möglichkeit damit aber durch eine erhöhte Anzahl von dedizierten Bedienelementen an dem Instrument, wodurch die Aufmerksamkeit eines operativ arbeitenden Chirurgen abgelenkt werden kann, die Bedienbarkeit der Geräte leidet und die Gefahr einer Fehlbedienung während einer laufenden Behandlung zunimmt.

Der Erfindung liegt daher als eine Aufgabe zugrunde, ein Verfahren und eine Einrichtung zur Steuerung einer Energiezufuhr zu einem medizinischen Instrument zu schaffen, die eine verbesserte Benutzersteuerung seiner Energieversorgung unter Verwendung der bereits an dem Instrument vorhandenen Komponenten erlaubt.

Darüber hinaus soll der Benutzer des medizinischen Instruments in die Lage versetzt werden, ohne zusätzliche Bedienelemente an dem Handstück eine von Betriebsarten einer Energieversorgung des medizinischen Instruments an dem Handstück auszuwählen.
Diese Aufgabe wird erfindungsgemäß durch eine Vorrichtung mit den Merkmalen des Anspruchs 1 und durch ein Verfahren mit den Merkmalen des Anspruchs 13 gelöst. Vorteilhafte Weiterbildungen sind Gegenstand der beigefügten Unteransprüche.
Der Erfindung liegt die allgemeine Idee zugrunde, an einer
Energieversorgungsvorrichtung für ein medizinisches Instrument, wie beispielsweise einem ein Handstück als das oder mit dem medizinische(n) Instrument mit Energie versorgenden Hochfrequenz-Generator, eine Generatorbetriebsart vorzusehen, welche eine Drück- und/oder Haltedauer eines Bedienelements an dem Handstück erfasst und auf der Grundlage der erfassten Dauer Änderungen an Parametern/ Parametereinstellungen der Energielieferung an das Handstück durchführt.
Diese allgemeine Idee wird nachstehend anhand einer beispielsweisen Vorrichtung zur bipolaren Gefäßversiegelung mit einem Handstück und einem das Handstück mit Hochfrequenzenergie versorgenden Hochfrequenz-Generator näher beschrieben.
An dem Hochfrequenz-Generator kann eine Generatorbetriebsart vorgesehen sein, welche die Drück- und/oder Haltedauer eines Hochfrequenzenergie zuschaltenden oder wegschaltenden Energiezufuhr-Bedienelements, beispielsweise ein Schalter oder ein Taster, an dem Handstück erfasst, um auf der Grundlage der erfassten Dauer Änderungen an Versiegelungsparametern (den Versiegelungsprozess am Behandlungsort beeinflussenden Parametern) vorzunehmen.
Teil der allgemeinen Idee ist zum einen, eine Steuerung an dem Instrument oder Handstück ohne jegliche zusätzliche Tasten oder Schalter zu ermöglichen und lediglich die bereits an dem (bestehenden) Instrument oder Handstück existierenden Komponenten des Instruments zu verwenden. Teil der vorstehenden allgemeinen Idee ist ferner, dass der Benutzer eine Betriebsart ohne zusätzliche Handbewegungen oder Schritte, welche den Ablauf oder Fluss einer Prozedur, einer Folge von Abläufen oder eines Behandlungsverlaufs unterbrechen, und ohne Kommunikation mit beispielsweise Klinikpersonal, auswählen und/oder umschalten bzw. wechseln kann. Ebenfalls Teil der vorstehenden allgemeinen Idee ist schließlich eine intuitive Lösung derart, dass ein Verfahren oder eine Vorgehensweise zur Festlegung bzw. Auswahl einer Betriebsart einem gewünschten Ergebnis ähnelt, d.h. ein gewünschtes Ergebnis bereits eine entsprechende Bedienung am Handstück zumindest impliziert.

Dazu kann beispielsweise bevorzugt eine weitere, beispielsweise dritte, Betriebsart zu den an dem Hochfrequenz-Generator bereits vorhandenen Betriebsarten hinzugefügt sein, die an dem Hochfrequenz-Generator aus- oder vorwählbar ist und nach Auswahl bzw. Vorwahl eine zusätzliche Funktionalität des bereits an dem Handstück vorhandenen Energiezufuhr-Bedienelements, das in diesem Fall beispielsweise als eine Art "Feuertaste" arbeiten kann, freigibt oder aktiviert.

Wenn diese Betriebsart an dem Hochfrequenz-Generator ausgewählt oder vorgewählt ist, kann dieser die Zeitdauer überwachen und/oder erfassen, während welcher das Energiezufuhr-Bedienelement an dem Handstück gedrückt gehalten wird.

Wird das Energiezufuhr-Bedienelement nur für eine vorbestimmte erste (kurze) Zeit oder Zeitdauer betätigt, erkennt der Hochfrequenz-Generator dies und liefert Energie entsprechend einer (zyklusbezogen kurzen) Normal- bzw. Standardbetriebsart (im beispielsweisen Fall einer Gefäßversiegelung einer kurz versiegelnden ersten oder normalen Betriebsart). Wird demgegenüber das Energiezufuhr-Bedienelement für eine vorbestimmte zweite (längere) Zeit oder Zeitdauer betätigt, beispielsweise für 0,8 Sekunden oder mehr (Größenordnung um eine Sekunde), erkennt die Energieversorgungsvorrichtung diese länger andauernde Betätigung und liefert Energie entsprechend einer (zyklusbezogen längeren) Betriebsart (im beispielsweisen Fall der Gefäßversiegelung der länger versiegelnden zweiten oder "Plus"-Betriebsart).

Alternativ oder zusätzlich kann diese weitere oder dritte Betriebsart, welche an dem Hochfrequenz-Generator gewählt wird, in einer variablen Änderung der Versiegelungsparameter resultieren. Beispielsweise kann vorgesehen sein, dass dann, wenn das Energiezufuhr-Bedienelement kurz gedrückt wird, eine "Standardenergiezufuhr" erfolgen, beispielsweise für eine "Standardversiegelung" als einer Grundversiegelungsbetriebsart. Je länger das Energiezufuhr-Bedienelement gedrückt wird, kann darüber hinaus eine zunehmend stärkere Änderung oder Anpassung der Versiegelungsprozessparameter erfolgen.

In Übereinstimmung mit der vorstehenden allgemeinen Idee sind verschiedenartige Konfigurationen bezüglich dessen, wie der Hochfrequenz-Generator die Energiezufuhr zu dem Handstück oder Instrument durchführt und wann er damit beginnt, darstellbar.

In einer beispielhaft möglichen Konfiguration kann der Hochfrequenz-Generator warten, bis ermittelt ist, welcher von Energielieferzyklen bzw. Energieabgabezyklen über das Handstück angefordert wird, und dann den angeforderten Zyklus beginnen.

In einer beispielhaft möglichen weiteren Konfiguration kann der Hochfrequenz-Generator unmittelbar mit Parametern beginnen, die einer Standardbetriebsart entsprechen bzw. zugeordnet sind, sobald das Energiezufuhr-Bedienelement betätigt (gedrückt) wird, und sodann den Prozess oder Zyklus auf veränderte Parameter, beispielsweise solche der Plus-Betriebsart wie vorstehend erwähnt, aktualisieren, nachdem oder falls er eine entsprechende Notwendigkeit ermittelt. In diesem Fall wird angenommen, dass die Zeit, die zur Ermittlung oder Bestimmung einer gewünschten Betriebsart benötigt wird, hinreichend kurz ist und sich bezüglich der Parameteränderung nicht signifikant auf den Versiegelungsprozessablauf auswirkt. In Abhängigkeit von Parametern kann der Hochfrequenz-Generator dazu ausgelegt sein, Prozessdaten für mehrere, beispielsweise zwei, Versiegelungsprozesse mitzuführen und zu verarbeiten, bis endgültig über eine tatsächlich durchzuführende Betriebsart entschieden ist.
Darüber hinaus kann diese Betriebsart so ausgelegt sein, dass sie eine veränderliche Wirkung auf die Versiegelungsparameter hat. Beispielsweise kann die Dauer, während welcher das Energiezufuhr-Bedienelement (gedrückt) gehalten wird, dazu verwendet werden, mehr als zwei variable Wirkungen auf den Ablauf des Versiegelungsprozesses zu zeigen. In einer praktischen Ausführungsform kann dann, wenn es für lediglich näherungsweise eine Sekunde (gedrückt) gehalten wird, entweder ein sanfter oder ein schrittweiser Anstieg in Versiegelungsparametern vorgesehen sein, und kann dann, wenn es länger (gedrückt) gehalten wird, diese Wirkung verstärkt bzw. beschleunigt werden.

Im Einzelnen wird die Aufgabe gelöst durch eine Energiezufuhr-Steuerungsvorrichtung eines medizinischen Instruments, die dazu angepasst ist, in Abhängigkeit einer Auswahl durch einen Bediener eine Energieversorgungseinrichtung des medizinischen Instruments in mehreren unterschiedlichen Energiezufuhr-Betriebsarten zu betreiben, wobei die Energiezufuhr-Steuerungsvorrichtung zumindest eine Betätigungserfassungs-Betriebsart beinhaltet, die dazu angepasst ist, eine oder mehrere Eigenschaften einer manuellen Betätigung eines an dem medizinischen Instrument angeordneten Energiezufuhr-Bedienelements und/oder eines Instrumenten-Bedienelements zu erfassen und auf der Grundlage der erfassten Betätigungseigenschaft(en) die Energieversorgungseinrichtung in einer ersten Betriebsart oder einer hierzu unterschiedlichen zweiten Betriebsart oder vorzugsweise einer weiteren, hierzu unterschiedlichen Betriebsart zu betreiben.

In anderen Worten wird die Aufgabe gelöst durch eine Vorrichtung zur Steuerung der Energiezufuhr zu einem medizinischen Instrument, mit dem medizinischen Instrument; und einer Energieversorgungseinrichtung, die dazu vorgesehen ist, das medizinische Instrument mit Energie zu versorgen, wobei die Energieversorgungseinrichtung eine Betätigungserfassungs-Betriebsart aufweist, die dazu vorgesehen ist, eine Betätigung eines an dem medizinischen Instrument angeordneten Energiezufuhr-Bedienelements zu erfassen und auf der Grundlage der erfassten Betätigung die Energieversorgung zu dem medizinischen Instrument in einer ersten Betriebsart oder einer zweiten Betriebsart durchzuführen.

Es ist erfindungsgemäß vorgesehen, dass an der Energieversorgungseinrichtung die erste Betriebsart und/ oder die zweite Betriebsart und die dritte Betriebsart/ die Betätigungserfassungs-Betriebsart direkt manuell vorwählbar sind, welche sich jeweils unterscheiden und dazu vorgesehen sind, das medizinische Instrument entsprechend der vorgewählten Energiezufuhr-Betriebsart mit Energie zu versorgen. Wenn beispielsweise die erste Betriebsart und die dritte Betriebsart vorwählbar sind, kann die zweite Betriebsart lediglich über ein entsprechendes/ geeignetes, vorzugsweise langes, Betätigen des Energiezufuhr-Bedienelements in der dritten Betriebsart ausgewählt und ausgeführt werden. Sind lediglich die zweite Betriebsart und die dritte Betriebsart vorwählbar, kann die erste Betriebsart lediglich über ein entsprechendes/ geeignetes, vorzugsweise kurzes, Betätigen des Energiezufuhr-Bedienelements in der dritten Betriebsart ausgewählt und ausgeführt werden. Es sei angeführt, dass, wenn nur die dritte Betriebsart/ die Betätigungserfassungs-Betriebsart vorwählbar ist, sowohl die erste als auch die zweite Betriebsart nur über ein geeignetes Betätigen des Energiezufuhr-Bedienelements ausgewählt und ausgeführt werden können.

Alternativ oder zusätzlich wird die Aufgabe gelöst durch eine Vorrichtung zur Steuerung der Energiezufuhr zu einem medizinischen Instrument, beinhaltend: das medizinische Instrument; und eine Energieversorgungseinrichtung, die zumindest eine erste und eine zweite an der Energieversorgungseinrichtung vorwählbare Energiezufuhr-Betriebsart aufweist und dazu vorgesehen ist, das medizinische Instrument entsprechend einer vorgewählten Energiezufuhr-Betriebsart mit Energie zu versorgen. Die Energieversorgungseinrichtung weist eine sich von den zumindest zwei Energiezufuhr-Betriebsarten unterscheidende und an der Energieversorgungseinrichtung vorwählbare dritte Betriebsart/ Betätigungserfassungs-Betriebsart auf, die dazu vorgesehen ist, eine Betätigung eines an dem medizinischen Instrument angeordneten Energiezufuhr-Bedienelements zu erfassen und auf der Grundlage der erfassten Betätigung die Energieversorgung zu dem medizinischen Instrument in der ersten oder der zweiten Betriebsart durchzuführen.

In der Praxis kann das medizinische Instrument ein bipolares Gefäßversiegelungsinstrument mit einem Werkzeug und einem Handstück sein, an dem ein Bedienelement zum Beginnen und/oder Beenden der Energiezufuhr zu dem Instrument angeordnet ist, und kann die Energieversorgungseinrichtung ein Hochfrequenz-Generator sein, der zur Ausgabe von Hochfrequenzenergie in einer von zumindest zwei unterschiedlichen Betriebsarten sein. Dabei kann die erste Betriebsart eine standardmäßig voreingestellte Betriebsart mit einer ersten Hochfrequenzenergieabgabe und die zweite Betriebsart eine wählbare Betriebsart mit einer zweiten, gegenüber der ersten Hochfrequenzenergieabgabe höheren Energieabgabe sein, und kann die dritte Betriebsart eine Betriebsart sein, die entsprechend einer von einer Zeitmesseinrichtung oder einem Zähler in der Energieversorgungseinrichtung gemessenen oder gezählten Zeit des Drückens oder Gedrückthaltens des Bedienelements die erste oder die zweite Betriebsart einstellt oder einsteuert, gemäß der sodann die eigentliche Hochfrequenzenergieabgabe an das medizinische Instrument erfolgt.

Bevorzugt ist die Energieversorgungseinrichtung dazu konfiguriert, in der dritten Betriebsart/ der Betätigungserfassungs-Betriebsart eine Zeitdauer einer Betätigung des Energiezufuhr-/ Instrumenten-Bedienelements als die Betätigungseigenschaft und/oder eine Betätigungskraft auf das Energiezufuhr-/ Instrumenten-Bedienelement zu erfassen und in Abhängigkeit von der erfassten Zeitdauer die Energieversorgung zu dem medizinischen Instrument in der ersten oder der zweiten Betriebsart durchzuführen. Vor dem Hintergrund einer zu schaffenden Verbesserung einer bestehenden Anordnung erlaubt die Hinzufügung einer dritten und insoweit übergeordneten Betriebsart zu bereits bestehenden zumindest zwei Betriebsarten eine übersichtlich darstellbare funktionelle Erweiterung der bestehenden Anordnung unter Entfall von weitreichenden Änderungen oder Eingriffen bezüglich der funktionell und/oder mechanisch bereits bestehenden Konfiguration.

Bevorzugt ist die erste Betriebsart eine standardmäßig voreingestellte Betriebsart, die dazu vorgesehen ist, eine Energieversorgung des medizinischen Instruments auf der Grundlage erster Parametereinstellungen durchzuführen, und ist die zweite Betriebsart eine über eine Betriebsartenwahleinrichtung an der Energieversorgungseinrichtung wählbare Betriebsart mit gegenüber der ersten Betriebsart veränderter Wirkung, die dazu vorgesehen ist, die Energieversorgung des medizinischen Instruments auf der Grundlage zweiter Parametereinstellungen durchzuführen. Dabei sind unter ersten und zweiten Parametereinstellungen sowohl das Einstellen unterschiedlicher Parameter als auch das Einstellen unterschiedlicher Werte für einzelne Parameter zu verstehen. Unter einer veränderten Wirkung der Parametereinstellungen sind unterschiedliche Ausprägungen der Versiegelung (Schnellversiegelung/ Heften oder endgültige Qualitätsversiegelung) und die damit einhergehenden Folgen (in Bezug auf Versiegelungsleistung und Entstehung von thermischen Gewebeschäden) zu verstehen. Diese zumindest zwei Betriebsarten erlauben bei beispielsweise einem Gefäßversiegelungssystem vorteilhaft einen kurzzeitig dauernden Versiegelungsprozess in der ersten Betriebsart und einen länger dauernden Versiegelungsprozess in der zweiten Betriebsart.

Bevorzugt ist die Energieversorgungseinrichtung dazu konfiguriert, in der dritten Betriebsart eine Betätigungszeitdauer des an dem medizinischen Instrument angeordneten Energiezufuhr-Bedienelements zu erfassen und die erfasste Betätigungszeitdauer mit einem vorbestimmten, insbesondere voreingestellten, Betätigungszeitdauerschwellwert derart abzugleichen, dass eine Energieversorgung des medizinischen Instruments in der ersten Betriebsart gewählt und ausgeführt wird, wenn die erfasste Betätigungszeitdauer kleiner als der Betätigungszeitdauerschwellwert ist, und eine Energieversorgung des medizinischen Instruments in der zweiten Betriebsart gewählt und ausgeführt wird, wenn die erfasste Betätigungszeitdauer größer oder gleich dem Betätigungszeitdauerschwellwert ist. In dieser Weise kann vorteilhaft eine an einem Handstück des medizinischen Instruments bereits angeordnete Feuertaste, mit welcher die Energieversorgungseinrichtung grundlegend für einen Beginn einer Energieabgabe und eine Beendigung einer Energieabgabe in der vorgewählten ersten oder zweiten Betriebsart steuerbar ist, in der vorgewählten dritten Betriebsart zusätzlich dazu genutzt werden, die erste oder die zweite Betriebsart an der Energieversorgungseinrichtung einzusteuern, je nachdem wie lange sie gedrückt bzw. gedrückt gehalten wird.

Bevorzugt liegt dabei der Betätigungszeitdauerschwellwert bei einer Zeitdauer von bis zu einer Sekunde, vorzugsweise zwischen 0,8 Sekunden und 1,0 Sekunden, weiter vorzugsweise bei genau 0,8 Sekunden. Es ist von Vorteil, wenn die Zeitdauer, während welcher die Bedientaste an dem medizinischen Instrument von einem Benutzer gedrückt gehalten werden soll, im Bereich von von dem Benutzer zur sicheren Aktivierung der ersten oder der zweiten Betriebsart gut zu unterscheidenden Zeitspannen liegt.

Bevorzugt ist dann, wenn die dritte Betriebsart an der Energieversorgungseinrichtung vorgewählt ist und die Energieversorgung des medizinischen Instruments in zumindest der zweiten Betriebsart durchgeführt wird, die Energieversorgungseinrichtung dazu konfiguriert, Parametereinstellungen der Energieversorgung des medizinischen Instruments in Abhängigkeit von einer erfassten Betätigungszeitdauer der Betätigung des Energiezufuhr-Bedienelements zu verändern und/oder anzupassen. Wird nicht nur das Verstreichen der ersten Betätigungszeitdauer zur Erkennung der Notwendigkeit der Durchführung der zweiten Betriebsart erfasst, sondern auch die Dauer der Betätigung des Bedienelements danach, können vorteilhaft Änderungen von Prozessparametern abgebildet und/oder beaufschlagt werden, die in Abhängigkeit von der tatsächlichen Dauer der Betätigung des Bedienelements variieren, beispielsweise ansteigen oder abnehmen bzw. größer oder kleiner werden.

Bevorzugt ist die Energieversorgungseinrichtung dazu vorgesehen, in der dritten Betriebsart einen über das Energiezufuhr-Bedienelement des medizinischen Instruments angeforderten Energielieferzyklus gemäß der ersten oder zweiten Betriebsart zu ermitteln und danach den angeforderten Energielieferzyklus zu beginnen. In einer solchen Ausführungsform wird vorteilhaft abgewartet, bis die für die erste Betriebsart vorgesehene Zeitdauer verstrichen ist, und bestimmt, ob das Bedienelement auch darüber hinaus gedrückt gehalten und die zweite Betriebsart angefordert wird oder nicht. In dieser Weise kann zuverlässig die von dem Benutzer tatsächlich gewünschte Betriebsart bereitgestellt werden.

Alternativ oder zusätzlich bevorzugt kann die Energieversorgungseinrichtung dazu konfiguriert sein, nach Erfassen einer ersten Betätigung des Energiezufuhr-Bedienelements einen ersten Energielieferzyklus in der ersten Betriebsart als einer Standardbetriebsart zu beginnen und bei Erfassen einer fortgesetzten Betätigung des Energiezufuhr-Bedienelements mit einer Dauer, die der zweiten Betriebsart zugeordnet ist, auf einen zweiten Energielieferzyklus mit der zweiten Betriebsart entsprechenden Parametereinstellungen zu wechseln. In einer solchen Ausführungsform wird vorteilhaft nicht abgewartet, bis die für die erste Betriebsart vorgesehene Zeitdauer verstrichen ist, sondern eine Entscheidung über die Anforderung der ersten oder der zweiten Betriebsart, welche andernfalls frühestens erst nach Verstreichen der für die erste Betriebsart vorgesehenen Zeitdauer getroffen werden könnte, nachgelagert. In dieser Weise kann die Energiezufuhr sofort in einer Grundversorgungsbetriebsart begonnen und das medizinische Instrument verzögerungsfrei mit Energie versorgt werden.

Es wird angemerkt, dass Weiterbildungen der beiden vorgenannten Arten bzw. Alternativen auch als die dritte und eine weitere an der Energieversorgungseinrichtung vorwählbare vierte Betriebsart realisiert sein können, oder die dritte Betriebsart über eine vorbestimmte Folge von (voreinstellenden) Betätigungen des Bedienelements an dem Handstück entsprechend umschaltbar sein kann. Beispielsweise kann eine Auslegung derart sein, dass ein vorbestimmt mehrmaliges Drücken des Bedienelements kurz hintereinander zwischen einem sofortigen Beginn der Energiezufuhr und einem verzögerten Beginn der Energiezufuhr hin und her wechselnd umschaltet.

Bevorzugt ist zumindest dann, wenn der verzögerte Beginn der Energiezufuhr gewählt ist, die Energieversorgungseinrichtung dazu konfiguriert, Prozessdaten für den ersten Energielieferzyklus und den zweiten Energielieferzyklus zumindest solange parallel zu führen und zu verarbeiten, bis auf Grundlage der erfassten Dauer eine tatsächlich durchzuführende Betriebsart festlegbar ist. Auf diese Weise wird vorteilhaft eine verzögerungsfreie Festlegung der tatsächlichen Betriebsart nach Verstreichen der ersten Zeitdauer unterstützt.

Bevorzugt ist die Energieversorgungseinrichtung dazu vorgesehen, in der dritten Betriebsart bei Durchführung der ersten und/oder der zweiten Betriebsart Prozessparameter kontinuierlich oder stufenweise zu erhöhen oder zu verringern, und bei Durchführung der zweiten Betriebsart die Prozessparameter stärker als in der ersten Betriebsart zu erhöhen oder zu verringern.

Bevorzugt ist das medizinische Instrument ein bipolares Gefäßversiegelungsinstrument und ist die Energieversorgungseinrichtung ein Hochfrequenz-Generator zur Versorgung des bipolaren Gefäßversiegelungsinstruments mit Hochfrequenzenergie.

Ein Gefäßversiegelungsinstrument und ein Hochfrequenz-Generator können vorteilhaft zumindest Teil eines bipolaren Gefäßversiegelungssystems bilden, das eine Vorrichtung wie vorstehend kurz beschrieben beinhaltet.

Außerdem bevorzugt beinhaltet ein Verfahren zum Einstellen von Betriebsarten zur Steuerung der Energiezufuhr von einer Energieversorgungseinrichtung zu einem medizinischen Instrument in zumindest einer ersten oder einer zweiten Energiezufuhr-Betriebsart die Schritte des Ermittelns, ob an der Energieversorgungseinrichtung eine dritte Betriebsart vorgewählt ist, falls die dritte Betriebsart vorgewählt ist, Ermitteln, ob ein Energiezufuhr-Bedienelement an dem medizinischen Instrument betätigt ist, und falls das Energiezufuhr-Bedienelement betätigt ist, Durchführen der Energieversorgung zu dem medizinischen Instrument in der ersten oder der zweiten Betriebsart auf der Grundlage der erfassten Betätigung und
Erfassen, ob an der Energieversorgungseinrichtung (10) die erste Betriebsart (B1) und/oder die zweite Betriebsart (B2) oder die Betätigungserfassungs-Betriebsart als eine dritte Betriebsart (B3), welche sich jeweils unterscheiden, direkt manuell vorgewählt ist, und Versorgen des medizinischen Instruments (20) entsprechend der vorgewählten Energiezufuhr-Betriebsart mit Energie.

Bevorzugt wird bei dem Verfahren in der dritten Betriebsart eine Zeitdauer einer Betätigung des Energiezufuhr-Bedienelements erfasst und in Abhängigkeit von der erfassten Zeitdauer die Energieversorgung zu dem medizinischen Instrument in der ersten oder der zweiten Betriebsart durchgeführt.

In dem Verfahren kann vorteilhaft die erste Betriebsart eine standardmäßig voreingestellte Betriebsart sein, die dazu vorgesehen ist, eine Energieversorgung des medizinischen Instruments auf der Grundlage erster Parametereinstellungen durchzuführen, und kann die zweite Betriebsart eine über eine Betriebsartenwahleinrichtung an der Energieversorgungseinrichtung wählbare Betriebsart mit gegenüber der ersten Betriebsart veränderter Wirkung sein, die dazu vorgesehen ist, die Energieversorgung des medizinischen Instruments auf der Grundlage zweiter Parametereinstellungen durchzuführen.

Bevorzugt kann das Verfahren dazu konfiguriert sein, in der dritten Betriebsart eine Betätigungszeitdauer des an dem medizinischen Instrument angeordneten Energiezufuhr-Bedienelements zu erfassen und die erfasste Betätigungszeitdauer mit einem vorbestimmten, insbesondere voreingestellten Betätigungszeitdauerschwellwert derart abzugleichen, dass eine Energieversorgung des medizinischen Instruments in der ersten Betriebsart gewählt und ausgeführt wird, wenn die erfasste Betätigungszeitdauer kleiner als der Betätigungszeitdauerschwellwert ist, und eine Energieversorgung des medizinischen Instruments in der zweiten Betriebsart gewählt und ausgeführt wird, wenn die erfasste Betätigungszeitdauer größer oder gleich dem Betätigungszeitdauerschwellwert ist.

Vorteilhaft kann dabei der Betätigungszeitdauerschwellwert bei einer Zeitdauer von bis zu einer Sekunde, vorzugsweise zwischen 0,8 Sekunden und 1,0 Sekunden, weiter vorzugsweise bei genau 0,8 Sekunden, liegen.

Weiter bevorzugt kann das Verfahren dann, wenn die dritte Betriebsart an der Energieversorgungseinrichtung vorgewählt ist und die Energieversorgung des medizinischen Instruments in der zweiten Betriebsart durchgeführt wird, in der dritten Betriebsart Parametereinstellungen der Energieversorgung des medizinischen Instruments in Abhängigkeit von einer erfassten Dauer der Betätigung des Energiezufuhr-Bedienelements verändern und/oder anpassen.

Bevorzugt kann ferner das Verfahren in der dritten Betriebsart einen über das Energiezufuhr-Bedienelement des medizinischen Instruments angeforderten Energielieferzyklus gemäß der ersten oder zweiten Betriebsart ermitteln und danach den angeforderten Energielieferzyklus beginnen.

Alternativ oder zusätzlich bevorzugt kann darüber hinaus das Verfahren nach Erfassen einer ersten Betätigung des Energiezufuhr-Bedienelements einen ersten Energielieferzyklus in der ersten Betriebsart als einer Standardbetriebsart beginnen und bei Erfassen einer fortgesetzten Betätigung des Energiezufuhr-Bedienelements mit einer Dauer, die der zweiten Betriebsart zugeordnet ist, auf einen zweiten Energielieferzyklus mit der zweiten Betriebsart entsprechenden Parametern wechseln.

Vorteilhaft kann das Verfahren dazu konfiguriert sein, Prozessdaten für den ersten Energielieferzyklus und den zweiten Energielieferzyklus zumindest solange parallel zu führen und zu verarbeiten, bis auf Grundlage der erfassten Dauer eine tatsächlich durchzuführende Betriebsart festlegbar ist.

Außerdem vorteilhaft kann das Verfahren in der dritten Betriebsart bei Durchführung der ersten und/oder der zweiten Betriebsart Prozessparameter kontinuierlich oder stufenweise erhöhen oder verringern, und bei Durchführung der zweiten Betriebsart die Prozessparameter stärker als in der ersten Betriebsart erhöhen oder verringern.

Die vorstehende Kurzbeschreibung zu Zwecken einer ersten Übersicht ist in keiner Weise beschränkend. Die Erfindung ist in verschiedenartigen Vorrichtungen und Verfahren anwendbar, bei welchen während eines laufenden Vorgangs an einem ersten Gerät verfügbare Betriebsarten von einem entfernten Bedienteil aus ohne zusätzliche Komponenten an dem Bedienteil beeinflussbar sein sollen.

Die Erfindung wird nachstehend anhand eines bevorzugten Ausführungsbeispiels unter Bezugnahme auf die beigefügte Zeichnung näher beschrieben. Soweit nicht anders angegeben, bezeichnen in der Zeichnung gleiche Bezugszeichen jeweils gleiche Komponenten, die nicht redundant beschrieben werden. Es zeigen:
Fig. 1 anhand einer schematischen Darstellung eines bipolaren Gefäßversiegelungssystems ein Ausführungsbeispiel einer Vorrichtung zur Steuerung der Energiezufuhr zu einem medizinischen Instrument; und
Fig. 2 ein Ablaufdiagramm eines beispielhaften Betriebsablaufs in dem Ausführungsbeispiel nach Fig. 1.

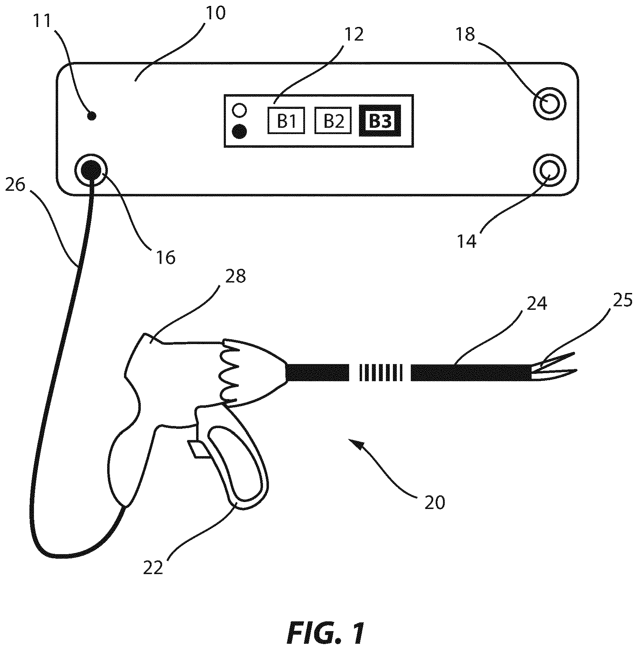

Fig. 1 zeigt schematisch ein bipolares Gefäßversiegelungssystem, in dem ein Ausführungsbeispiel einer Vorrichtung zur Steuerung der Energiezufuhr zu einem medizinischen Instrument verkörpert ist. Es wird angemerkt, dass eine Grundform eines solchen bipolaren Gefäßversiegelungssystems mit zwei Betriebsarten wie eingangs beschrieben als Aesculap® Caiman® (medizinisches Instrument) und Aesculap® Lektrafuse® (Hochfrequenz-Generator) verfügbar und insoweit bekannt ist. Insoweit bekannte Elemente und Komponenten werden daher nicht redundant beschrieben.

Gemäß Fig. 1 besteht das bipolare Gefäßversiegelungssystem somit aus zumindest einem Hochfrequenz-Generator 10 und einem bipolaren Gefäßversiegelungsinstrument 20, das elektrisch mit dem Hochfrequenz-Generator 10 verbunden ist.

Der Hochfrequenz-Generator 10 ist in einem Gehäuse angeordnet, das im Wesentlichen Hochfrequenzenergie erzeugende Elektronik und Verarbeitungselektronik einschließlich einer CPU, zugeordnetem Speicher, Eingabe/Ausgabe-Abschnitten und dergleichen, einen Netzanschluss (rückseitig; nicht dargestellt), einen Netzschalter (rückseitig; nicht dargestellt), zumindest eine Signalisierungsleuchte 11, eine Anzeigevorrichtung 12, einen Anschluss für einen Fußbedienschalter 14, einen Anschluss 16 für zumindest ein medizinisches Instrument und einen Betriebsartenwahlschalter 18 aufweist oder aufnimmt. Der Hochfrequenz-Generator 10 bildet in dieser Form eine Energieversorgungsvorrichtung oder -einrichtung zur Versorgung des bipolaren Gefäßversiegelungsinstruments 20 mit hochfrequenter Energie.

Das Gefäßversiegelungsinstrument 20 als medizinisches Instrument umfasst ein Handstück 22 und ein stab- oder schaftförmiges Werkzeug 24. Das Werkzeug 24 kann ein artikulierbares distales Ende aufweisen, an dem eine Gefäßversiegelungseinrichtung bzw. ein Gefäßversiegelungsabschnitt beispielsweise mit zwei zueinander verschwenkbaren und mit HF-Elektroden versehenen Branchen 25 zur Versiegelung von zumindest Gefäßen während eines chirurgischen Eingriffs und ggf. eine Schneideeinrichtung bzw. ein Schneideabschnitt zur Ausführung von Schnitten an Gewebe und/oder Gefäßen angeordnet sind. Die Gefäßversiegelungseinrichtung komprimiert das Gewebe und überträgt Wärme, welche aus der dem Instrument 20 zugeführten hochfrequenten Energie bzw. Hochfrequenz generiert wird, auf das zu versiegelnde Gewebe oder Gefäß.

Das Gefäßversiegelungsinstrument 20 ist mittels eines Kabels 26 mit dem Anschluss 16 des Hochfrequenz-Generators 10 verbindbar und weist an seinem Handstück 22 wenigstens ein Betätigungs- oder Bedienelement 28 auf, über welches durch Betätigung oder Bedienung eine Energiezufuhr von dem Hochfrequenz-Generator 10 angefordert werden kann und das daher als ein Energiezufuhr-Bedienelement 28 arbeitet. Das Energiezufuhr-Bedienelement 28 kann wie in Fig. 1 dargestellt als Taster/ Betätigungsknopf bereitgestellt sein, jedoch sind auch andere Ausführungsformen darstellbar.

In diesem Ausführungsbeispiel sind an dem Hochfrequenz-Generator 10 wenigstens drei Betriebsarten B1, B2, B3 für den Versiegelungsprozess, d.h. eine erste (B1), eine zweite (B2) und eine dritte Betriebsart (B3), bereitgestellt und an dem Betriebsartenwahlschalter 18 vorwählbar und/oder wechselbar, wenn der Hochfrequenz-Generator 10 eingeschaltet ist.

Eine Konfiguration des Hochfrequenz-Generators 10 kann dabei derart sein, dass eine gewählte Betriebsart bis zum Ausschalten des Hochfrequenz-Generators 10 erhalten bleibt, während eines Eingriffs geändert werden kann, und unabhängig von einem jeweils angeschlossenen Instrument ist. Eine eingestellte Betriebsart kann auf der Anzeigevorrichtung 12 angezeigt und mittels beispielsweise eines Rahmens und/oder fetter Schriftdarstellung als ausgewählt gekennzeichnet werden. Außerdem können verschiedenartige Rückmeldungen des Hochfrequenz-Generators 10 über Lichtsignalisierungen an Leuchtmitteln oder Anzeigesegmenten der Anzeigevorrichtung 12 und/oder Tonsignalisierungen wie beispielsweise Pieptöne vorgesehen sein. Beispielsweise können unterschiedliche Betriebsarten durch unterschiedliche Blinkfrequenzen von Signalisierungsleuchten oder Anzeigesegmenten und/oder unterschiedliche Frequenzen von Pieptönen während einer Hochfrequenzabgabe unterscheidbar gemacht sein.

Nachstehend wird ein grundlegender Betriebsablauf an dem Hochfrequenz-Generator 10 kurz beschrieben. Der grundlegende Betriebsablauf beinhaltet das Verbinden des Hochfrequenz-Generators 10 mit dem elektrischen Stromnetz, das Einschalten des Netzschalters (eine zugeordnete Signallampe leuchtet auf), das Durchführen eines automatischen Selbsttests, und nach dessen Beendigung und Bereitschaft des Geräts die Wahl einer gewünschten Betriebsart. Danach können ein Fußschalter, sofern vorhanden, und das bipolare Gefäßversiegelungsinstrument 20 an den Hochfrequenz-Generator 10 angeschlossen und mit dem Taster bzw. Energiezufuhr-Bedienelement 28 oder gegebenenfalls dem (nicht gezeigten) Fußschalter aktiviert werden.

In einer Grundkonfiguration, die der bekannten Anordnung entspricht, kann beispielsweise der Versiegelungsprozess (die HF-Abgabe) durch einmaliges Drücken des Energiezufuhr-Bedienelements 28 gestartet und durch nochmaliges Drücken desselben beendet oder abgebrochen werden. Ohne nochmaliges Drücken des Energiezufuhr-Bedienelements stoppt der Hochfrequenz-Generator 10 die HF-Abgabe nach Beendigung des Versiegelungsprozesses automatisch. Das heißt, dass in der bekannten Grundkonfiguration die zu verwendende Betriebsart vor Beginn einer Behandlung oder eines Eingriffs zwingend am Hochfrequenz-Generator 10 vorzuwählen ist, falls nicht eine nach dem Einschalten des Hochfrequenz-Generators 10 voreingestellte Betriebsart beibehalten werden soll, und das Energiezufuhr-Bedienelement 28 an dem Handstück 22 lediglich Beginn und gegebenenfalls Ende einer HF-Abgabe in der am Hochfrequenz-Generator 10 vorgewählten Betriebsart triggert.

Gemäß dem vorliegenden Ausführungsbeispiel ist eine weitere vorwählbare, dritte Betriebsart B3 bereitgestellt. Die dritte Betriebsart B3 ist eine Betriebsart, in welcher der Hochfrequenz-Generator 10 erfasst und ermittelt, ob und wie lange das Energiezufuhr-Bedienelement 28 an dem Handstück 22 gedrückt bzw. gedrückt gehalten wird. In Übereinstimmung mit dem Ergebnis der Erfassung bzw. Ermittlung einer entsprechenden Zeitdauer schaltet der Hochfrequenz-Generator 10 auf eine der beiden HF-Abgabe-Betriebsarten B1, B2 um und/oder ändert Parameter, die die HF-Abgabe beeinflussen und sich auf den Versiegelungsprozess auswirken können.

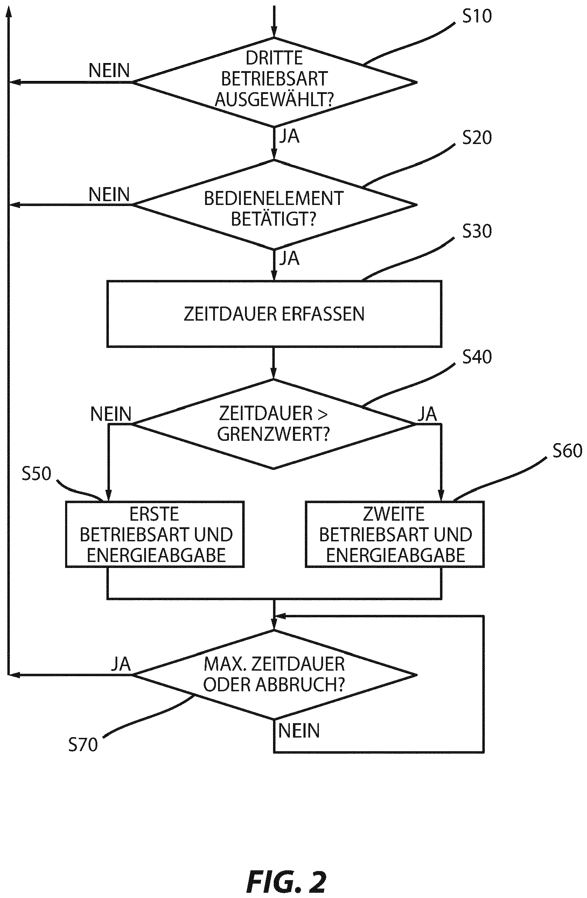

Nachstehend wird ein Betriebsablauf unter Verwendung der dritten Betriebsart B3 unter Bezugnahme auf Fig. 2 näher beschrieben. Eine Beschreibung des grundlegenden Betriebsablaufs wie vorstehend erwähnt ist in Fig. 2 nicht redundant wiederholt. In anderen Worten sei angenommen, dass für den in Fig. 2 wiedergegebenen Ablauf der Hochfrequenz-Generator 10 betriebsbereit, das Gefäßversiegelungselement 20 funktionsfähig mit diesem verbunden ist und eine gültige Voreinstellung oder Vorwahl einer Betriebsart vorliegt.

In S10 prüft der Hochfrequenz-Generator 10, ob die dritte Betriebsart B3 vorgewählt ist. Falls die dritte Betriebsart B3 nicht vorgewählt ist (NEIN in S10), kehrt der Ablauf zu einem übergeordneten Prozessteil zurück, aus welchem er beispielsweise zyklisch wieder S10 als Eintrittspunkt in die Verarbeitung nach Fig. 2 erreichen kann. Falls die dritte Betriebsart B3 vorgewählt ist (JA in S10), schreitet die Verarbeitung zu S20 fort.

In S20 prüft der Hochfrequenz-Generator 10, ob das Energiezufuhr-Bedienelement 28 an dem Handstück 22 betätigt bzw. gedrückt ist. Falls das Energiezufuhr-Bedienelement 28 nicht gedrückt ist (NEIN in S20), kehrt der Ablauf ebenfalls zu dem übergeordneten Prozessteil zurück. Falls das Energiezufuhr-Bedienelement 28 gedrückt ist (JA in S20), schreitet die Verarbeitung zu S30 fort.

In S30 beginnt der Hochfrequenz-Generator 10 unter Verwendung beispielsweise eines Zeitgebers oder Zeitzählers, eine Zeitdauer oder Zeitspanne zu erfassen, während welcher das Energiezufuhr-Bedienelement 28 betätigt oder gedrückt gehalten wird. Danach schreitet die Verarbeitung zu S40 fort. Zuvor kann der Zeitgeber geeignet initialisiert werden und/oder sein letzter Zustand bzw. Zählwert gepuffert oder zu beispielsweise Protokollierungszwecken in einem Speicher des Hochfrequenz-Generators 10 gespeichert werden.

In S40 ermittelt der Hochfrequenz-Generator 10, ob die erfasste Zeitdauer kürzer oder länger als ein vorbestimmter Grenzwert/ Schwellwert ist. Falls die erfasste Zeitdauer kürzer als der vorbestimmte Grenzwert ist, schreitet die Verarbeitung zu S50 fort. Falls die erfasste Zeitdauer länger als der vorbestimmte Grenzwert ist, schreitet die Verarbeitung zu S60 fort.

In S50 legt der Hochfrequenz-Generator 10 für die HF-Abgabe an das Gefäßversiegelungsinstrument 20 die erste Betriebsart B1 fest, die einer Standardbetriebsart mit ersten Parametern für eine Energiezufuhr zugunsten eines kurzen Versiegelungsprozesses entspricht, und gibt die entsprechende HF-Energie an das Gefäßversiegelungsinstrument 20 aus. Nachdem die Ausgabe der HF-Energie an das Gefäßversiegelungsinstrument 20 begonnen ist, schreitet die Verarbeitung zu S70 fort.

In S60 legt der Hochfrequenz-Generator 10 für die HF-Abgabe an das Gefäßversiegelungsinstrument 20 die zweite Betriebsart B2 fest, die einer Betriebsart mit gegenüber der ersten Betriebsart B1 erhöhter HF-Abgabe und mit zweiten Parametern für eine Energiezufuhr zugunsten eines langen Versiegelungsprozesses entspricht, und gibt die entsprechende HF-Energie an das Gefäßversiegelungsinstrument 20 aus. Nachdem die Ausgabe der HF-Energie an das Gefäßversiegelungsinstrument 20 begonnen ist, schreitet die Verarbeitung zu S70 fort.

In S70 prüft der Hochfrequenz-Generator 10, ob eine maximale Dauer der Energieabgabe an das Gefäßversiegelungsinstrument 20 erreicht ist, und/oder ob das Gedrückthalten des Energiezufuhr-Bedienelements 28 an dem Handstück 22 beendet ist. In anderen Worten prüft der Hochfrequenz-Generator 10, ob eine Abbruchbedingung für die laufende HF-Abgabe eingetreten ist oder nicht. Falls ermittelt wird, dass die Abbruchbedingung eingetreten ist (JA in S70), beendet der Hochfrequenz-Generator 10 die Zeiterfassung und die laufende Energieabgabe, und kehrt die Verarbeitung zu dem übergeordneten Prozessteil zurück, aus welchem wieder S10 als Eintrittspunkt in die Verarbeitung nach Fig. 2 erreicht werden kann. Falls ermittelt wird, dass die Abbruchbedingung nicht eingetreten ist (NEIN in S70), wird die Abfrageverarbeitung gemäß S70 wiederholt.

In einer Modifikation des vorliegenden Ausführungsbeispiels kann nach beispielsweise S50 und/oder S60 ein Prozessteil mit zumindest einem zusätzlichen Schritt S55 und/oder S65 vorgesehen sein, in welchem Parameter (Versiegelungsparameter) der laufenden HF-Abgabe in Abhängigkeit von einer aktuell erfassten Zeitdauer aktualisiert, verändert und/oder angepasst werden.

Beispielsweise kann in der dritten Betriebsart B3 vorgesehen sein, in zumindest einer der Betriebsarten B1 und B2, vorzugsweise der zweiten Betriebsart B2, die Versiegelungsparameter ausgehend von geeigneten Anfangswerten umso stärker zu verändern, je länger das Energiezufuhr-Bedienelement 28 gedrückt gehalten wird, oder die Versiegelungsparameter ab einer vorbestimmten Gedrückthaltedauer beginnend zu verändern oder nicht weiter zu verändern, oder für eine vorbestimmte Zeitdauer ansteigend oder absteigend zu verändern oder eine Veränderung zu pausieren.

Falls vorgesehen ist, in mehreren der Betriebsarten die Versiegelungsparameter zeitdauerabhängig zu verändern, kann der Hochfrequenz-Generator 10 Prozessdaten für die mehreren Versiegelungsbetriebsarten in Verarbeitung führen bzw. halten, bis entsprechend der erfassten Zeitdauer über die festzulegende Versiegelungsbetriebsart endgültig entschieden ist, und eine mehrvariable Beeinflussung, beispielsweise eine zweifach variable Beeinflussung, des Versiegelungsprozesses durchführen. In diesem Fall kann beispielsweise dann, wenn das Energiezufuhr-Bedienelement 28 für etwa eine Sekunde gehalten wird, eine weiche oder schrittweise Zunahme einer Änderung in bzw. an Versiegelungsparametern durchgeführt werden, und dann, wenn es für eine längere Zeitdauer gehalten wird, diese Wirkung oder Beeinflussung verstärkt, beispielsweise beschleunigt, werden.

In einer weiteren Modifikation des vorliegenden Ausführungsbeispiels kann vor beispielsweise S30 zumindest ein Schritt S25 vorgesehen sein, in welchem unmittelbar nach Erfassen der Betätigung des Energiezufuhr-Bedienelements 28 die HF-Abgabe in der ersten Betriebsart B1 (Standardbetriebsart) begonnen wird. In diesem Fall kann in dem nachfolgenden Schritt S40 eine Beibehaltung oder ein Wechsel der Betriebsart (beispielsweise von der Betriebsart B1 auf die Betriebsart B2) entsprechend der im weiteren Verlauf tatsächlich erfassten Zeitdauer erfolgen.

Insgesamt ist somit in diesem Ausführungsbeispiel eine Generatorbetriebsart beschrieben, welche die Dauer eines Drückens/Haltens eines Bedienelements oder einer Bedientaste (RF-Taste) misst, um Änderungen an den Versiegelungsparametern zu bewirken, d.h. auf der Grundlage der gemessenen Dauer Parameteränderungen bezüglich der HF-Abgabe einzusteuern.

Wie vorstehend beschrieben wurde, beinhaltet eine Vorrichtung zur Steuerung der Energiezufuhr zu einem medizinischen Instrument das medizinische Instrument (Gefäßversiegelungsinstrument 20) und eine Energieversorgungseinrichtung (Hochfrequenz-Generator 10), die zumindest eine erste und eine zweite an der Energieversorgungseinrichtung vorwählbare Energiezufuhr-Betriebsart (Betriebsarten B1 und B2) aufweist und dazu angeordnet ist, das medizinische Instrument entsprechend einer vorgewählten Energiezufuhr-Betriebsart mit Energie zu versorgen. Die Vorrichtung ist dadurch gekennzeichnet, dass die Energieversorgungseinrichtung weiter eine sich von den zumindest zwei Energiezufuhr-Betriebsarten unterscheidende und an der Energieversorgungseinrichtung vorwählbare dritte Betriebsart (Betriebsart B3) aufweist, die dazu angeordnet ist, eine Betätigung eines an dem medizinischen Instrument angeordneten Energiezufuhr-Bedienelements (Griffstück oder RF-Taste 28) zu erfassen und auf der Grundlage der erfassten Betätigung die Energieversorgung zu dem medizinischen Instrument in der ersten oder der zweiten Betriebsart durchzuführen.

Die Erfindung wurde vorstehend anhand eines bevorzugten Ausführungsbeispiels beschrieben. Es versteht sich, dass Einzelheiten des beschriebenen bevorzugten Ausführungsbeispiels die Erfindung als solche nicht beschränken und sich für den Fachmann naheliegend verschiedenartige Änderungen, Modifikationen und/oder Äquivalente ergeben können, die als solche allesamt im Rahmen der durch die beigefügten Ansprüche definierten Schutzbereich der Erfindung liegen.

## Patentansprüche

1. Energiezufuhr-Steuerungsvorrichtung eines medizinischen Instruments (20), die dazu angepasst ist, in Abhängigkeit einer Auswahl durch einen Bediener eine Energieversorgungseinrichtung (10) des medizinischen Instruments (20) in mehreren unterschiedlichen Energiezufuhr-Betriebsarten zu betreiben, wobei die Energiezufuhr-Steuerungsvorrichtung zumindest eine Betätigungserfassungs-Betriebsart beinhaltet, die dazu angepasst ist, eine oder mehrere Eigenschaften einer manuellen Betätigung eines an dem medizinischen Instrument (20) angeordneten Energiezufuhr-Bedienelements (28) und/oder eines Instrumenten-Bedienelements zu erfassen und auf der Grundlage der erfassten Betätigungseigenschaft(en) die Energieversorgungseinrichtung (20) in einer ersten Betriebsart (B1) oder einer hierzu unterschiedlichen zweiten Betriebsart (B2) oder vorzugsweise einer weiteren, hierzu unterschiedlichen Betriebsart (Bx) zu betreiben, **dadurch gekennzeichnet, dass** an der Energieversorgungseinrichtung (10) die erste Betriebsart (B1) und/oder die zweite Betriebsart (B2) und die Betätigungserfassungs-Betriebsart als eine dritte Betriebsart (B3) direkt manuell vorwählbar sind, welche sich jeweils unterscheiden und dazu vorgesehen sind, das medizinische Instrument (20) entsprechend der vorgewählten Energiezufuhr-Betriebsart mit Energie zu versorgen.

2. Vorrichtung nach Anspruch 1, **dadurch gekennzeichnet, dass** die Energieversorgungseinrichtung (10) dazu konfiguriert ist, in der Betätigungserfassungs-Betriebsart eine Zeitdauer einer Betätigung des Energiezufuhr-/ Instrumenten-Bedienelements als die Betätigungseigenschaft und/oder eine Betätigungskraft auf das Energiezufuhr-/ Instrumenten-Bedienelement zu erfassen und in Abhängigkeit von der erfassten Zeitdauer die Energieversorgung zu dem medizinischen Instrument (20) in der ersten oder der zweiten Betriebsart (B1, B2) durchzuführen.

3. Vorrichtung nach Anspruch 1 oder 2, **dadurch gekennzeichnet, dass** die erste Betriebsart (B1) eine standardmäßig voreingestellte Betriebsart ist, die dazu vorgesehen ist, eine Energieversorgung des medizinischen Instruments (20) auf der Grundlage erster Parametereinstellungen durchzuführen, und die zweite Betriebsart (B2) eine über eine Betriebsartenwahleinrichtung an der Energieversorgungseinrichtung (10) wählbare Betriebsart mit gegenüber der ersten Betriebsart (B1) veränderter Wirkung ist, die dazu vorgesehen ist, die Energieversorgung des medizinischen Instruments (20) auf der Grundlage zweiter Parametereinstellungen durchzuführen.

4. Vorrichtung nacheinem der Ansprüche 1 bis 3, **dadurch gekennzeichnet, dass** die Energieversorgungseinrichtung (10) dazu konfiguriert ist, in der dritten Betriebsart (B3) eine Betätigungszeitdauer des an dem medizinischen Instrument (20) angeordneten Energiezufuhr-Bedienelements (28) zu erfassen und die erfasste Betätigungszeitdauer mit einem vorbestimmten, insbesondere voreingestellten, Betätigungszeitdauerschwellwert derart abzugleichen, dass eine Energieversorgung des medizinischen Instruments (20) in der ersten Betriebsart (B1) gewählt und ausgeführt wird, wenn die erfasste Betätigungszeitdauer kleiner als der Betätigungszeitdauerschwellwert ist, und eine Energieversorgung des medizinischen Instruments (20) in der zweiten Betriebsart (B2) gewählt und ausgeführt wird, wenn die erfasste Betätigungszeitdauer größer oder gleich dem Betätigungszeitdauerschwellwert ist.

5. Vorrichtung nach Anspruch 4, **dadurch gekennzeichnet, dass** der Betätigungszeitdauerschwellwert bei einer Zeitdauer von bis zu einer Sekunde, vorzugsweise zwischen 0,8 Sekunden und 1,0 Sekunden, weiter vorzugsweise bei genau 0,8 Sekunden, liegt.

6. Vorrichtung nach einem der Ansprüche 1 bis 5, **dadurch gekennzeichnet, dass** dann, wenn die dritte Betriebsart (B3) an der Energieversorgungseinrichtung (10) vorgewählt ist und die Energieversorgung des medizinischen Instruments (20) in der zweiten Betriebsart (B2) durchgeführt wird, die Energieversorgungseinrichtung dazu konfiguriert ist, Parametereinstellungen der Energieversorgung des medizinischen Instruments (20) in Abhängigkeit von einer erfassten Betätigungszeitdauer der Betätigung des Energiezufuhr-Bedienelements (28) zu verändern und/oder anzupassen.

7. Vorrichtung nach einem der Ansprüche 1 bis 6, **dadurch gekennzeichnet, dass** die Energieversorgungseinrichtung (10) dazu vorgesehen ist, in der dritten Betriebsart (B3) einen über das Energiezufuhr-Bedienelement (28) des medizinischen Instruments (20) angeforderten Energielieferzyklus gemäß der ersten oder zweiten Betriebsart (B1, B2) zu ermitteln und danach den angeforderten Energielieferzyklus zu beginnen.

8. Vorrichtung nach einem der Ansprüche 1 bis 7, **dadurch gekennzeichnet, dass** die Energieversorgungseinrichtung (10) dazu konfiguriert ist, nach Erfassen einer ersten Betätigung des Energiezufuhr-Bedienelements (28) einen ersten Energielieferzyklus in der ersten Betriebsart (B1) als einer Standardbetriebsart zu beginnen und bei Erfassen einer fortgesetzten Betätigung des Energiezufuhr-Bedienelements mit einer Dauer, die der zweiten Betriebsart (B2) zugeordnet ist, auf einen zweiten Energielieferzyklus mit der zweiten Betriebsart (B2) entsprechenden Parametereinstellungen zu wechseln.

9. Vorrichtung nach Anspruch 8, **dadurch gekennzeichnet, dass** die Energieversorgungseinrichtung (10) dazu konfiguriert ist, Prozessdaten für den ersten Energielieferzyklus und den zweiten Energielieferzyklus zumindest solange parallel zu führen und zu verarbeiten, bis auf Grundlage der erfassten Dauer eine tatsächlich durchzuführende Betriebsart festlegbar ist.

10. Vorrichtung nach einem der Ansprüche 1 bis 9, **dadurch gekennzeichnet, dass** die Energieversorgungseinrichtung (10) dazu vorgesehen ist, in der dritten Betriebsart (B3) bei Durchführung der ersten und/oder der zweiten Betriebsart (B1, B2) Prozessparameter kontinuierlich oder stufenweise zu erhöhen oder zu verringern, und bei Durchführung der zweiten Betriebsart (B2) die Prozessparameter stärker als in der ersten Betriebsart (B1) zu erhöhen oder zu verringern.

11. Vorrichtung nach einem der vorangehenden Ansprüche, bei der das medizinische Instrument (20) ein bipolares Gefäßversiegelungsinstrument ist und die Energieversorgungseinrichtung (10) ein Hochfrequenz-Generator zur Versorgung des bipolaren Gefäßversiegelungsinstruments mit Hochfrequenzenergie ist.

12. Bipolares Gefäßversiegelungssystem mit einer Vorrichtung nach einem der vorangehenden Ansprüche.

13. Verfahren zum Einstellen von Betriebsarten zur Steuerung der Energiezufuhr von einer Energieversorgungseinrichtung (10) zu einem medizinischen Instrument (20) in zumindest einer Betätigungserfassungs-Betriebsart, mit den Schritten:
Ermitteln, ob an der Energieversorgungseinrichtung (10) die Betätigungserfassungs-Betriebsart vorgewählt ist;
falls die Betätigungserfassungs-Betriebsart vorgewählt ist, Ermitteln, ob ein Energiezufuhr-Bedienelement (28) an dem medizinischen Instrument (20) betätigt ist; und
falls das Energiezufuhr-Bedienelement (28) betätigt ist, Erfassen einer oder mehrerer Eigenschaften der Betätigung des Energiezufuhr-Bedienelements (28) und Durchführen der Energieversorgung zu dem medizinischen Instrument (20) in einer ersten Betriebsart (B1) oder einer zweiten Betriebsart (B2) auf der Grundlage der erfassten Betätigungseigenschaft(en) und,
Erfassen, ob an der Energieversorgungseinrichtung (10) die erste Betriebsart (B1) und/oder die zweite Betriebsart (B2) oder die Betätigungserfassungs-Betriebsart als eine dritte Betriebsart (B3), welche sich jeweils unterscheiden, direkt manuell vorgewählt ist, und Versorgen des medizinischen Instruments (20) entsprechend der vorgewählten Energiezufuhr-Betriebsart mit Energie.

14. Verfahren nach Anspruch 13, **dadurch gekennzeichnet, dass** in der Betätigungserfassungs-Betriebsart eine Zeitdauer einer Betätigung des Energiezufuhr-Bedienelements (28) erfasst und in Abhängigkeit von der erfassten Zeitdauer die Energieversorgung zu dem medizinischen Instrument (20) in der ersten oder der zweiten Betriebsart (B1, B2) durchgeführt wird.

## Claims

1. Energy supply controlling device of a medical instrument (20) which is suitable to be operated in a plurality of different energy supply operating modes depending on the selection of an energy supply device (10) of the medical instrument (20) by an operator,
wherein
the energy supply controlling device has at least one actuating detection operating mode which is suitable to detect one or a plurality of properties of a manual actuation of an energy supply operating element (28) arranged on the medical instrument (20) and/or of an instrument operating element and on the basis of the detected actuation property(ies) to operate the energy supply device (20) in a first operating mode (B1) or a second operating mode (B2) which is different to this or preferably another operating mode (Bx) which is different to this, **characterised in that**
on the energy supply device (10) the first operating mode (B1) and/or the second operating mode (B2) and the actuation detection operating mode as a third operating mode (B3) can be preselected manually which differ from one another and are provided to supply the medical instrument (20) with energy depending on the selected energy supply operating mode.

2. Device according to claim 1, **characterised in that** the energy supply device (10) is configured such that in the actuation detection operating mode a duration of an actuation of the energy supply/instrument operating element as the actuation property and/or an actuation force on the energy supply/instrument operating element is detected and depending on the detected duration, the energy supply to the medical instrument (20) is carried out in the first or the second operating mode (B1, B2).

3. Device according to claim 1 or 2, **characterised in that** the first operating mode (B1) is a standard preset operating mode which is provided to carry out energy supply of the medical instrument (20) on the basis of the first parameter settings and the second operating mode (B2) is an operating mode with a different effect compared to the first operating mode (B1) which can be selected via an operating mode selection device on the energy supply device (10) which is provided to carry out the energy supply of the medical instrument (20) on the basis of the second parameter settings.

4. Device according to any one of claims 1 to 3, **characterised in that** the energy supply device (10) is configured such that in the third operating mode (B3) an actuation duration of the energy supply operating element (28) arranged on the medical instrument (20) is detected and the detected actuation duration is compared to a predetermined, in particular preset, actuation duration threshold value such that an energy supply of the medical instrument (20) is selected in the first operating mode (B1) and is carried out if the selected actuation duration is below the actuation duration threshold value and an energy supply of the medical instrument (20) is selected in the second operating mode (B2) and is carried out if the selected actuation duration is above the actuation duration threshold value.

5. Device according to claim 4, **characterised in that** the actuation duration threshold value for a duration is up to one second, preferably between 0.8 seconds and 1.0 seconds, more preferably exactly 0.8 seconds.

6. Device according to any one of claims 1 to 5, **characterised in that** if the third operating mode (B3) is preselected on the energy supply device (10) and the energy supply of the medical instrument (20) is carried out in the second operating mode (B2), the energy supply device is configured such that parameter settings of the energy supply of the medical instrument (20) are changed and/or adapted depending on a detected actuation duration of the actuation of the energy supply operating element (28).

7. Device according to any one of claims 1 to 6, **characterised in that** the energy supply device (10) is provided such that in the third operating mode (B3) an energy supply cycle requested via the energy supply operating element (28) of the medical device (20) according to the first or second operating mode (B1, B2) is determined and then the requested energy supply cycle is begun.

8. Device according to any one of claims 1 to 7, **characterised in that** the energy supply device (10) is configured such that after detection of a first actuation of the energy supply operating element (28) a first energy supply cycle is begun in the first operating mode (B1) as a standard operating mode and on detection of a continued actuation of the energy supply operating element with a duration which is assigned to the second operating mode (B2) this is changed to a second energy supply cycle with the second operating mode (B2) corresponding to the parameter settings.

9. Device according to claim 8, **characterised in that** the energy supply device (10) is configured to run and to process process data for the first energy supply cycle and the second energy supply cycle at least as long as parallel until, on the basis of the detected duration, it is possible to determine an operating mode to actually be carried out.

10. Device according to any one of claims 1 to 9, **characterised in that** the energy supply device (10) is provided such that in the third operating mode (B3) when carrying out the first and/or second operating mode (B1, B2) process parameters are continuously or gradually increased or reduced and when carrying out the second operating mode (B2) the process parameter is increased or reduced more than in the first operating mode (B1).

11. Device according to any one of the preceding claims, in which the medical instrument (20) is a bipolar vessel sealing instrument and the energy supply device (10) is a high-frequency generator for supplying the bipolar vessel sealing instrument with high-frequency energy.

12. Bipolar vessel sealing system with a device according to any one of the preceding claims.

13. Method for adjusting operating modes for controlling the energy supply from an energy supply device (10) to a medical instrument (20) in at least one actuation detection operating mode, with the following steps:
Determining whether the actuation detection operating mode is selected on the energy supply device (10);
If the actuation detection operating mode is selected, determining whether an energy supply operating element (28) is actuated on the medical instrument (20); and
If the energy supply operating element (28) is actuated, detecting one or a plurality of properties of the actuation of the energy supply operating element (28) and carrying out energy supply to the medical instrument (20) in a first operating mode (B1) or a second operating mode (B2) on the basis of the detected actuation property(ies); and
Detecting whether the first operating mode (B1) and/or the second operating mode (B2) or the actuation detection operating mode as a third operating mode (B3), which each differ from one another, is directly manually preselected on the energy supply device (10); and
Supplying the medical instrument (20) with energy depending on the selected energy supply operating mode.

14. Method according to claim 13, **characterised in that** in the actuation detection operating mode a duration of an actuation of the energy supply operating element (28) is detected and depending on the detected duration energy supply to the medical instrument (20) is carried out in the first or the second operating mode (B1, B2).

## Revendications

1. Dispositif pour commander l'alimentation en énergie d'un instrument médical (20), qui est adapté pour faire fonctionner un dispositif d'alimentation en énergie (10) de l'instrument médical (20) dans plusieurs modes de fonctionnement d'alimentation en énergie différents en fonction d'une sélection effectuée par un opérateur,
dans lequel
le dispositif pour commander l'alimentation en énergie contient au moins un mode de fonctionnement de détection d'actionnement, qui est adapté pour détecter une ou plusieurs propriétés d'un actionnement manuel d'un élément de commande d'alimentation en énergie (28) agencé au niveau de l'instrument médical (20) et/ou d'un élément de commande d'instrument et pour faire fonctionner le dispositif d'alimentation en énergie (20) dans un premier mode de fonctionnement (B1) ou un deuxième mode de fonctionnement (B2) différent de celui-ci ou de préférence un autre mode de fonctionnement (Bx) différent de celui-ci, sur la base de la(des) propriété(s) d'actionnement détectée(s), **caractérisé en ce que**
le premier mode de fonctionnement (B1) et/ou le deuxième mode de fonctionnement (B2) et le mode de fonctionnement de détection d'actionnement en tant que troisième mode de fonctionnement (B3) sont présélectionnables directement manuellement au niveau du dispositif d'alimentation en énergie (10), lesquels se distinguent respectivement et sont prévus pour alimenter l'instrument médical (20) en énergie conformément au mode de fonctionnement d'alimentation en énergie présélectionné.

2. Dispositif selon la revendication 1, **caractérisé en ce que** le dispositif d'alimentation en énergie (10) est configuré pour détecter dans le mode de fonctionnement de détection d'actionnement une durée d'un actionnement de l'élément de commande d'alimentation en énergie/d'instrument en tant que la propriété d'actionnement et/ou une force d'actionnement sur l'élément de commande d'alimentation en énergie/d'instrument et pour réaliser l'alimentation en énergie à l'instrument médical (20) dans le premier ou le deuxième mode de fonctionnement (B1, B2) en fonction de la durée détectée.

3. Dispositif selon la revendication 1 ou 2, **caractérisé en ce que** le premier mode de fonctionnement (B1) est un mode de fonctionnement préréglé par défaut, qui est prévu pour réaliser une alimentation en énergie de l'instrument médical (20) sur la base de premiers réglages de paramètres, et le deuxième mode de fonctionnement (B2) est un mode de fonctionnement sélectionnable par le biais du dispositif de sélection de mode de fonctionnement au niveau du dispositif d'alimentation en énergie (10) avec effet modifié par rapport au premier mode de fonctionnement (B1), qui est prévu pour réaliser l'alimentation en énergie de l'instrument médical (20) sur la base de deuxièmes réglages de paramètres.

4. Dispositif selon l'une quelconque des revendications 1 à 3, **caractérisé en ce que** le dispositif d'alimentation en énergie (10) est configuré pour détecter dans le troisième mode de fonctionnement (B3) une durée d'actionnement de l'élément de commande d'alimentation en énergie (28) agencé au niveau de l'instrument médical (20) et de comparer la durée d'actionnement détectée avec une valeur seuil de durée d'actionnement prédéterminée, en particulier préréglée, de sorte qu'une alimentation en énergie de l'instrument médical (20) est sélectionnée et réalisée dans le premier mode de fonctionnement (B1), lorsque la durée d'actionnement détectée est inférieure à la valeur seuil de durée d'actionnement, et une alimentation en énergie de l'instrument médical (20) est sélectionnée et réalisée dans le deuxième mode de fonctionnement (B2), lorsque la durée d'actionnement détectée est supérieure ou égale à la valeur seuil de durée d'actionnement.

5. Dispositif selon la revendication 4, **caractérisé en ce que** la valeur seuil de durée d'actionnement est pour une durée allant jusqu'à une seconde, de préférence entre 0,8 seconde et 1,0 seconde, de préférence encore exactement 0,8 seconde.

6. Dispositif selon l'une quelconque des revendications 1 à 5, **caractérisé en ce qu'**alors, lorsque le troisième mode de fonctionnement (B3) est présélectionné au niveau du dispositif d'alimentation en énergie (10) et l'alimentation en énergie de l'instrument médical (20) est réalisée dans le deuxième mode de fonctionnement (B2), le dispositif d'alimentation en énergie est configuré pour modifier et/ou adapter des réglages de paramètres de l'alimentation en énergie de l'instrument médical (20) en fonction d'une durée d'actionnement détectée de l'actionnement de l'élément de commande d'alimentation en énergie (28).

7. Dispositif selon l'une quelconque des revendications 1 à 6, **caractérisé en ce que** le dispositif d'alimentation en énergie (10) est prévu pour déterminer dans le troisième mode de fonctionnement (B3) un cycle de fourniture d'énergie demandé par le biais de l'élément de commande d'alimentation en énergie (28) de l'instrument médical (20) conformément au premier ou deuxième mode de fonctionnement (B1, B2) et commencer ensuite le cycle de fourniture d'énergie demandé.

8. Dispositif selon l'une quelconque des revendications 1 à 7, **caractérisé en ce que** le dispositif d'alimentation en énergie (10) est configuré pour commencer après détection d'un premier actionnement de l'élément de commande d'alimentation en énergie (28), un premier cycle de fourniture d'énergie dans le premier mode de fonctionnement (B1) en tant que mode de fonctionnement standard et pour passer lors de la détection d'un actionnement poursuivi de l'élément de commande d'alimentation en énergie avec une durée, qui est associée au deuxième mode de fonctionnement (B2), à un deuxième cycle de fourniture d'énergie avec des réglages de paramètres correspondant au deuxième mode de fonctionnement (B2).

9. Dispositif selon la revendication 8, **caractérisé en ce que** le dispositif d'alimentation en énergie (10) est configuré pour amener et traiter les données de processus pour le premier cycle de fourniture d'énergie et le deuxième cycle de fourniture d'énergie en parallèle au moins jusqu'à ce qu'un mode de fonctionnement à réaliser effectivement sur la base des données saisies peut être fixé.

10. Dispositif selon l'une quelconque des revendications 1 à 9, **caractérisé en ce que** le dispositif d'alimentation en énergie (10) est prévu pour augmenter ou diminuer en continu ou graduellement des paramètres de processus dans le troisième mode de fonctionnement (B3) lors de la réalisation du premier et/ou du deuxième mode de fonctionnement (B1, B2), et pour augmenter ou diminuer les paramètres de processus davantage que dans le premier mode de fonctionnement (B1) lors de la réalisation du deuxième mode de fonctionnement (B2).

11. Dispositif selon l'une quelconque des revendications précédentes, dans lequel l'instrument médical (20) est un instrument de scellement vasculaire bipolaire et le dispositif d'alimentation en énergie (10) est un générateur haute fréquence pour l'alimentation de l'instrument de scellement vasculaire bipolaire en énergie haute fréquence.

12. Système de scellement vasculaire bipolaire avec un dispositif selon l'une quelconque des revendications précédentes.

13. Procédé de réglage de modes de fonctionnement pour la commande de l'alimentation en énergie d'un dispositif d'alimentation en énergie (10) à un instrument médical (20) dans au moins un mode de fonctionnement de détection d'actionnement, avec les étapes de :
détermination du fait si le mode de fonctionnement de détection d'actionnement est présélectionné au niveau du dispositif d'alimentation en énergie (10) ;
si le mode de fonctionnement de détection d'actionnement est présélectionné, détermination du fait si un élément de commande d'alimentation en énergie (28) est actionné au niveau de l'instrument médical (20) ; et
si l'élément de commande d'alimentation en énergie (28) est actionné, détection d'une ou de plusieurs propriétés de l'actionnement manuel de l'élément de commande d'alimentation en énergie (28) et réalisation de l'alimentation en énergie à l'instrument médical (20) dans un premier mode de fonctionnement (B1) ou un deuxième mode de fonctionnement (B2) sur la base de la(des) propriété(s) d'actionnement détectée(s) et,
détection si le premier mode de fonctionnement (B1) et/ou le deuxième mode de fonctionnement (B2) ou le mode de fonctionnement de détection d'actionnement en tant que troisième mode de fonctionnement (B3) est présélectionné directement manuellement au niveau du dispositif d'alimentation en énergie (10) et alimentation de l'instrument médical (20) en énergie conformément au mode de fonctionnement d'alimentation en énergie présélectionné.

14. Procédé selon la revendication 13, **caractérisé en ce que** dans le mode de fonctionnement de détection d'actionnement, une durée d'un actionnement de l'élément de commande d'alimentation en énergie (28) est détectée et en fonction de la durée détectée, l'alimentation en énergie à l'instrument médical (20) est réalisée dans le premier ou le deuxième mode de fonctionnement (B1, B2).
